# EUROPÄISCHE PATENTSCHRIFT

(11) **EP 3 496 968 B1**
(45) Veröffentlichungstag und Bekanntmachung des Hinweises auf die Patenterteilung: **14.05.2025**
(21) Anmeldenummer: 17761798.2
(22) Anmeldetag: 09.08.2017
(51) Int. Cl.: B60K 28/00, G01N 33/497, G16H 40/63, G16H 40/67

(54) **VORRICHTUNG, SUBSTANZMESSGERÄT, VERFAHREN UND COMPUTERPROGRAMM ZUR IDENTIFIKATION EINES SUBSTANZMESSGERÄTES**
DEVICE, SUBSTANCE MEASURING DEVICE, METHOD AND COMPUTER PROGRAM FOR IDENTIFYING A SUBSTANCE MEASURING DEVICE
DISPOSITIF, DISPOSITIF DE MESURE DE SUBSTANCE, PROCÉDÉ ET PROGRAMME INFORMATIQUE POUR IDENTIFIER UN DISPOSITIF DE MESURE DE SUBSTANCE

(30) Priorität: 15.08.2016 DE 102016009834
(43) Veröffentlichungstag der Anmeldung: 19.06.2019
(73) Patentinhaber: Dräger Safety AG & Co. KGaA, 23560 Lübeck (DE)
(72) Erfinder: MORLEY, Stefan, 23556 Lübeck (DE); SARCINELLI, Alexander, 22926 Ahrensburg (DE); WILLNER, Matthias, 23564 Lübeck (DE)
(74) Vertreter: Guthöhrlein, Gerhard
(86) Internationale Anmeldenummer: PCT/EP2017/000966
(87) Internationale Veröffentlichungsnummer: WO 2018/033240

(56) Entgegenhaltungen:
- EP-A1- 2 075 151
- EP-A1- 2 127 599
- EP-A1- 2 237 034
- WO-A1-2012/041505
- WO-A1-2017/064023
- US-A1- 2008 170 762
- US-A1- 2011 186 632
- US-A1- 2015 204 844
- US-A1- 2015 360 696
- US-B1- 6 748 792
- INTOXIMETERS: "Trademark/Service Mark Application, Principal Register ALCO-SENSOR FST STANDARD CHARACTERS YES USPTO-GENERATED IMAGE ALCO-SENSOR FST United States LEGAL ENTITY INFORMATION TYPE corporation STATE/COUNTRY OF INCORPORATION Missouri GOODS AND/OR SERVICES AND BASIS INFORMATION BREATH ALCOHOL ANALYZERS AN", 8 July 2008 (2008-07-08), pages 1 - 7, XP093045700, Retrieved from the Internet <URL:https://tsdr.uspto.gov/documentviewer?caseId=sn77245548&docId=PRC20080603030447#docIndex=10&page=1> [retrieved on 20230510]
- ANONYMOUS: "Notice of publication Serial number 77/245,548", 18 June 2008 (2008-06-18), pages 1 - 1, XP093045701, Retrieved from the Internet <URL:https://tsdr.uspto.gov/documentviewer?caseId=sn77245548&docId=NOP20080618081828#docIndex=8&page=1> [retrieved on 20230510]

## Beschreibung

Ausführungsbeispiele beziehen sich auf eine Vorrichtung, ein Substanzmessgerät, ein Verfahren, ein Computerprogramm, zur Identifikation eines Substanzmessgerätes, insbesondere aber nicht ausschließlich, auf eine optische Kennung zur Identifikation des Substanzmessgerätes.

In der konventionellen Technik sind verschiedene Konzepte bekannt, die beispielsweise im Falle von Alkohol- und/oder Drogensucht eine Überwachung der Patienten außerhalb von speziell dafür vorgesehenen Einrichtungen erlauben. Im Rahmen von mobilem Personal können Hausbesuche durchgeführt werden, bzw. können die Patienten durch regelmäßiges Erscheinen und Abgabe von Proben ambulant überwacht werden. Dies zieht entsprechenden Aufwand auf der Seite der Patienten bzw. auf Seiten des Pflegepersonals nach sich. Im Folgenden wird das Beispiel der Alkoholsucht stellvertretend für Alkohol-, Drogen- oder Sucht allgemein und damit verbundene Überwachungsstrategien für die jeweiligen Substanzen betrachtet.

Z.B. wird mit Atemalkoholbestimmung die Messung des Alkoholgehaltes in der Atemluft bezeichnet. Im Falle von Drogenüberwachung können Speichelproben als geeignetes Mittel zur Überwachung eines Konsums und/oder einer Abstinenz eines Patienten dienen. Beispielsweise nach Genuss alkoholhaltiger Getränke oder Lebensmittel findet in den Lungenbläschen ein Gasaustausch zwischen der Atemluft und dem aufgenommenen Alkohol statt. Der im peripheren Blut enthaltene Alkohol wird von der eingeatmeten Frischluft aufgenommen und mit der Ausatmungsluft abgegeben, wodurch eine Messung erfolgen kann, die Rückschlüsse auf die Blutalkoholkonzentration zulässt.

Für die Bestimmung des Atemalkohols können Handgeräte (mobile Geräte) sowie stationäre Geräte genutzt werden. Durch eine Messung auf elektrochemischem oder physikalischem Weg wird der Atemalkoholwert ermittelt und auf einer Anzeigeeinheit (meist Bildschirme/Displays) dargestellt.

Das Hauptanwendungsgebiet der Atemalkoholbestimmung sind Verkehrskontrollen im Straßenverkehr durch die Polizei. Allerdings werden sogenannte "Alkoholtester" auch im privaten Gebrauch, im medizinischen Bereich (Suchtkliniken) oder in den Anwendungen "Alkohol-Zündschlosssperre" und "Homemonitoring" (Überwachung zu Hause) verwendet. In letzteren - den sogenannten "Offender (Täter)-Programmen" - wird häufig durch die Rechtsprechung ein Foto bei der Probegabe verlangt, um sicherzustellen, dass Probegeber und Offender die identische Person sind.

### BESTÄTIGUNGSKOPIE

Beispielsweise gibt es im Bereich Homemonitoring, ein Konzept, das eine Lösung über eine Kombination von Atemalkoholmessgerät und Smartphone (Mobilfunkendgerät) inklusive entsprechender Applikation (App) für das Smartphone vorsieht. In einigen Lösungen kann während der Probengabe ein Foto mit einer im Alkoholmessgerät integrierten Kamera getätigt werden.

EP 2 237 034 A1 offenbart eine Vorrichtung und ein Verfahren zur Erkennung einer korrekten Anwendung eines Alkoholmessgerätes von einer Testperson.

EP 2 127 599 A1 offenbart ein chemisches Messgerätsystem und ein Verfahren umfassend ein Videoüberwachungssystem zur Gesichtserkennung des Testers.

US 2015/360696 A1 offenbart ein Fahrzeugsicherheitssystem, das biometrische Daten nutzt.

WO 2017/064023 A1 offenbart ein System zur Detektion einer gasförmigen Substanz ausgeatmet durch den Nutzer.

US 2015/204844 A1 offenbart ein System zum Überwachen der Trunkenheit eines Nutzers.

Intoximeters: "Trademark/Service Mark Application, Principal Register ALCO-SENSOR FST STANDARD CHARACTERS YES USPTO-GENERATED IMAGE ALCO-SENSOR FST United States LEGAL ENTITY INFORMATION TYPE corporation STATE/COUNTRY OF INCORPORATION Missouri GOODS AND/OR SERVICES AND BASIS INFORMATION BREATH ALCOHOL ANALYZERS AN" offenbart einen Alkoholtestsensor, der einen Barcode und eine Seriennummer umfasst.

Es besteht daher ein Bedarf daran, ein verbessertes Konzept zur Überwachung eines Patienten zu schaffen.

Diesem Bedarf tragen die anhängigen unabhängigen Ansprüche Rechnung.

Ausführungsbeispiele basieren auf der Erkenntnis, dass im Bereich "Homemonitoring" eine zuverlässige Information darüber, ob der Probegeber und der Offender bei einem verlangten Atemtest/Substanztest die identische Person sind, hilfreich ist. Bei einem Homemonitoring-Programm kann es sich beispielsweise um eine Bewährungsauflage für Wiederholungstäter von Alkohol-/Drogenmissbrauch im Straßenverkehr oder im familiären Haushalt mit möglicher häuslicher Gewalt, handeln. Z.B. kann eine Bewährungsauflage randomisierte Atemalkoholtests oder Speichelprobentests während des Tags vorsehen. In manchen Ausführungsbeispielen kann eine Nachweisfunktion via Foto implementiert sein, sodass nachvollziehbar wird, ob der Probengeber auch der Wiederholungstäter ist.

Manche Beispiele sehen eine Kombination von Substanzmessgerät und Smartphone vor.

Auf dem Smartphone wird eine App installiert, mit der der Probegeber folgende Schritte durchführen kann:
1. Das Smartphone wird gestartet, das Atemalkoholgerät startet automatisch mit;
2. Der Probegeber startet die App über eine Schaltfläche auf dem Smartphone;
3. Eine Benutzeroberfläche fordert den Probanden auf, das Smartphone so auszurichten; dass das Gesicht des Probegebers durch die Frontkamera des Smartphones während der Probegabe fotografiert werden kann;
4. Es erscheint eine Rückmeldung, dass das Smartphone richtig positioniert ist;
5. Der Nutzer wird aufgefordert einen Atemalkoholtest abzugeben;
6. Der Nutzer betätigt eine Schaltfläche, um den Test zu starten;
7. Er pustet in das Gerät während er das Smartphone weiterhin gleichermaßen positioniert;
8. Während der Probegabe wird ein Foto getätigt;
9. Durch einen Hinweis, weiß der Probegeber, wann der Test abgeschlossen ist; und
10. Testergebnisse und Foto werden auf dem Smartphone gespeichert und an eine rechtlich befugte Person übermittelt.

In solchen Beispielen kann über händisches Sichten der vorhandenen Fotos nachgewiesen werden, dass der Probegeber auch wirklich die Person ist, die den Atemalkoholtest abgegeben hat. Diese Prüfung über händische Sichtung allein kann umfangreich und aufwendig sein. Die Möglichkeit durch Stichproben Verstöße zu ermitteln kann eher gering sein. Verstöße, wie zum Beispiel die Situation, dass der Probegeber ein Foto von sich macht, aber eine andere Person für sich pusten lässt, können nicht immer vermieden werden. Darüber hinaus gibt es eine Art der Manipulation, bei der der Straftäter ein neues Gerät kauft und in Folge die Probegabe durch eine dritte Person durchführen lässt und er selbst mit dem neugekauften Gerät lediglich eine Testabgabe simuliert und somit einwandfrei auf dem Foto mit Gerät erscheint. Ausführungsbeispiele basieren daher ferner auf dem Gedanken, das Substanzmessgerät identifizierbar zu machen.

Die Erfindung bezieht sich auf eine Vorrichtung zur eindeutigen und individuellen Identifikation eines Substanzmessgerätes eines Probanden, wobei die Vorrichtung eine optische Kennung umfasst, die ausgebildet ist zur Wiedererkennung des Substanzmessgerätes und zur Verifikation einer Zuordnung zwischen dem Substanzmessgerät und dem Probanden, wobei die optische Kennung einer Anordnung innerhalb eines Dreiecks von mehreren Markierungen, Barcodes, und/oder "Quick Response"-Codes, QR, oder von einer Kombination mindestens einer Markierung mit mindestens einem Barcode und/ oder mindestens einem QR-Code für ein Gehäuse des Substanzmessgerätes entspricht.

Es kann insofern eine Zuordnung eines Substanzgerätes zu einer Person erfolgen oder bereits durchgeführt worden sein, beispielsweise im Rahmen einer Überwachung oder eines Homemonitorings. In diesem Zusammenhang können auch die Begriffe "remote monitoring" (im Sinne von allgemeiner Fernüberwachung) oder "home alcohol monitoring" verwendet werden.

Die optische Kennung wird dann verwendet, um zu verifizieren, dass das Substanzgerät und der Probegeber einander zugeordnet oder miteinander verknüpft sind. Die Vorrichtung erlaubt so eine Überprüfung dieser Zuordnung oder Verbindung/Verknüpfung und erlauben eine Information darüber zu bestimmen, ob der Probegeber bei Abgabe der Probe auch das richtige, das dem Probegeber individuell zugeordnete Substanzmessgerät verwendet. Die Vorrichtung erlaubt so eine Verifikation einer Zuordnung zwischen einem Substanzmessgerät und einem Probanden. Die optische Kennung der Vorrichtung ist zur Verifikation oder Überprüfung einer Zuordnung zwischen dem Substanzmessgerät und dem Probanden ausgebildet. Zumindest manche Ausführungsbeispiele können durch die Verknüpfung zwischen Person/Proband und Substanzmessgerät eine verbesserte Erfolgsquote bei der Überwachung erzielen als dies beispielsweise bei einer Verknüpfung zwischen Substanzmessgerät und einem Ort oder einem Fahrzeug der Fall wäre.

Die optische Kennung kann dementsprechend an oder in dem Substanzmessgerät vorgesehen sein, um zumindest temporär eine Wiedererkennung des Substanzgeräts und so die Überprüfung der Zuordnung zwischen Substanzmessgerät und Probegeber zu ermöglichen. Die optische Kennung kann dabei permanent sein, beispielsweise als äußerliches Merkmal, oder auch temporär, beispielsweise kann ein optisches Signal über eine Lichtquelle oder ein Display verwendet werden. In manchen Ausführungsbeispielen kann die optische Kennung auch eineindeutig oder einzigartig sein, insofern, dass das Substanzmessgerät von allen anderen Substanzmessgeräten unterschieden werden kann. Generell wird der Begriff der "eindeutigen und individuellen" Identifikation hier jedoch nicht theoretisch, sondern eher praktisch verstanden und meint dabei, dass durch die optische Kennung das Substanzgerät mit einer gewissen Wahrscheinlichkeit, z.B. > 90%, 95%, 99%, 99,9%, 99,99% usw., als das dem Probegeber zugeordnete Substanzmessgerät wiedererkannt werden kann. In anderen Worten kann "eindeutige und individuelle" Identifikation auch dahingehend ausgelegt werden, dass ein einem Probegeber zugeordnetes Substanzmessgerät mittels der optischen Kennung mit einer Fehlerwahrscheinlichkeit von weniger als 10%, 1%, 0,1%, 0,01%, 0,001% usw. wiedererkannt werden kann. In Ausführungsbeispielen kann die Wiedererkennung mittels optischer Bilderfassung (Foto, Video) und digitaler Bilderverarbeitung erfolgen.

Dabei kann eine zu messende Substanz einem Atemalkohol und/oder Betäubungsmitteln im Speichel des Probanden entsprechen. Die optische Kennung umfasst eine Anordnung innerhalb eines Dreiecks von mehreren Markierungen, Barcodes, und/oder "Quick Response"-Codes, QR, oder von einer Kombination mindestens einer Markierung mit mindestens einem Barcode und/oder mindestens einem QR-Code für ein Gehäuse des Substanzmessgerätes Die Markierungen oder optischen Codes können bei der Identifikation des Substanzmessgerätes hilfreich sein. Die Anordnung entspricht einer Anordnung innerhalb eines Dreiecks. Eine dreieckige Anordnung kann Vorteile bei der Erkennung aus verschiedenen Blickwinkeln bieten, da ein Dreieck in seiner Form diesbezüglich relativ robust ist. So kann in manchen Ausführungsbeispielen die Anordnung zumindest drei Marker umfassen, die entlang der Seiten eines Dreiecks angeordnet sind.

In einigen weiteren Ausführungsbeispielen kann die optische Kennung ein oder mehrere Reflektoren umfassen. Reflektoren können Vorteile, insbesondere bei der elektronischen oder digitalen Detektion, bieten. Die optische Kennung kann in weiteren Ausführungsbeispielen ein oder mehrere reflektierende Folienabschnitte umfassen. Folienabschnitte bieten den Vorteil, dass diese einfach auf einem Gehäuse eines Substanzmessgerätes aufgebracht werden können oder montierbar sind.

In manchen Ausführungsbeispielen kann die optische Kennung eine eindeutige Kennung umfassen.

Insofern kann das Substanzmessgerät durch die optische Kennung eindeutig identifiziert werden. Die optische Kennung kann eine Sequenz optischer Signale umfassen, die durch ein oder mehrere an dem Substanzmessgerät angeordnete Lichtquellen erzeugbar ist. Insofern kann die optische Kennung in manchen Ausführungsbeispielen auch veränderbar bzw. von außen nicht direkt erkennbar sein. Die Lichtquellen können ausgebildet sein, um Licht in einem nicht sichtbaren Bereich zu emittieren. Insofern kann die Kennung nicht ohne weitere Hilfsmittel erkannt werden. Die Lichtquellen können ferner ausgebildet sein, um Licht in einem Infrarotbereich zu emittieren. Ausführungsbeispiele können so einfach implementierbar sein und es können auch im sichtbaren Lichtbereich nicht transparente Gehäuse verwendet werden, durch die mittels Infrarotlicht hindurchgeschienen werden kann. Die ein oder mehreren Lichtquellen können so durch das Gehäuse geschützt werden.

Die Erfindung bezieht sich auch auf ein Verfahren zur eindeutigen und individuellen Identifikation eines Substanzmessgerätes eines Probanden, wobei das Substanzmessgerät eine optische Kennung aufweist. Das Verfahren umfasst ein Durchführen einer Substanzmessung durch den Probanden und ein Erfassen von optischen Bilddaten des Probanden während der Substanzmessung zusammen mit dem Substanzmessgerät. Das Verfahren umfasst ferner ein Bestimmen von Information darüber, ob die optische Kennung in den Bilddaten detektierbar ist und ein Identifizieren des Substanzmessgerätes basierend auf der optischen Kennung. Das Verfahren umfasst ferner das Verifizieren einer Zuordnung zwischen dem Substanzmessgerät und dem Probanden, wobei die optische Kennung verwendet wird, die einer Anordnung innerhalb eines Dreiecks von ein oder mehreren Markierungen, Barcodes, und/oder einem "Quick Response"-Codes, QR, oder von einer Kombination mindestens einer Markierung mit mindestens einem Barcode und/oder mindestens einem QR-Code für ein Gehäuse des Substanzmessgerätes entspricht. In manchen Ausführungsbeispielen kann das Verfahren ferner ein Weiterleiten der Bilddaten zusammen mit der Information zur Überprüfung umfassen.

Beschrieben wird ferner eine Kamera, die ausgebildet ist, um eines der hierin beschriebenen Verfahren durchzuführen. In einigen Aspekten kann die Kamera ferner einen Speicher umfassen und ausgebildet sein, um die Bilddaten zusammen mit der Information abzuspeichern. Weiterhin beschrieben ist ein Mobilfunkgerät mit der Kamera gemäß obiger Beschreibung. Das Mobilfunkgerät kann in manchen Aspekten in seiner Funktion für die Identifikation des Substanzmessgerätes mit der Kamera ausgebildet sein und in seinen weiteren Funktionen eingeschränkt sein. In einigen weiteren Aspekten kann das Mobilfunkgerät ausgebildet sein, um Anrufe zu nicht mehr als drei vordefinierten Rufnummern durchführen zu können.

Ein weiteres Ausführungsbeispiel ist ein Computerprogramm zur Durchführung zumindest eines der oben beschriebenen Verfahren, wenn das Computerprogramm auf einem Computer, einem Prozessor oder einer programmierbaren Hardwarekomponente abläuft. Weiterhin beschrieben ist auch ein digitales Speichermedium, das maschinen- oder computerlesbar ist, und das elektronisch lesbare Steuersignale aufweist, die mit einer programmierbaren Hardwarekomponente so zusammenwirken können, dass eines der oben beschriebenen Verfahren ausgeführt wird.

Es folgen Beschreibungen der Zeichnungen. Es zeigen:
Fig. 1 ein Ausführungsbeispiel einer Vorrichtung zur Identifikation eines Substanzmessgerätes;
Fig. 2 ein weiteres Ausführungsbeispiel einer Vorrichtung zur Identifikation eines Substanzmessgerätes im Einsatz mit einem Probanden;
Fig. 3 Substanzmessgeräte mit verschiedenen Kennungen; und
Fig. 4 ein Blockschaltbild eines Ausführungsbeispiels eines Ablaufdiagramms eines Verfahrens zur Identifikation eines Substanzmessgerätes.

Bei der nachfolgenden Beschreibung der beigefügten Figuren, können gleiche Bezugszeichen gleiche oder vergleichbare Komponenten bezeichnen. Ferner können zusammenfassende Bezugszeichen für Komponenten und Objekte verwendet werden, die mehrfach in einem Beispiel oder in einer Zeichnung auftreten, jedoch hinsichtlich eines oder mehrerer Merkmale gemeinsam beschrieben werden. Komponenten oder Objekte, die mit gleichen oder zusammenfassenden Bezugszeichen beschrieben werden, können hinsichtlich einzelner, mehrerer oder aller Merkmale, beispielsweise ihrer Dimensionierungen, gleich, jedoch gegebenenfalls auch unterschiedlich ausgeführt sein, sofern sich aus der Beschreibung nicht etwas anderes explizit oder implizit ergibt. Optionale Komponenten sind in den Figuren mit gestrichelten Linien oder Pfeilen dargestellt. Gleiche Bezugszeichen bezeichnen in der gesamten Figurenbeschreibung gleiche oder ähnliche Elemente.

Man beachte, dass ein Element, das als mit einem anderen Element "verbunden" oder "verkoppelt" bezeichnet wird, mit dem anderen Element direkt verbunden oder verkoppelt sein kann oder dass dazwischenliegende Elemente vorhanden sein können. Wenn ein Element dagegen als "direkt verbunden" oder "direkt verkoppelt" mit einem anderen Element bezeichnet wird, sind keine dazwischenliegenden Elemente vorhanden. Andere Begriffe, die verwendet werden, um die Beziehung zwischen Elementen zu beschreiben, sollten auf ähnliche Weise interpretiert werden (z.B., "zwischen" gegenüber "direkt dazwischen", "angrenzend" gegenüber "direkt angrenzend" usw.). Wie hierin verwendet, sollen die Singularformen " einer," "eine", "eines " und "der, die, das" auch die Pluralformen beinhalten, solange der Kontext nicht eindeutig etwas anderes angibt. Ferner sei klargestellt, dass die Ausdrücke wie z.B. "beinhaltet", "beinhaltend", "aufweist", "umfasst", "umfassend" und/oder "aufweisend", wie hierin verwendet, das Vorhandensein von genannten Merkmalen, ganzen Zahlen, Schritten, Arbeitsabläufen, Elementen und/oder Komponenten angeben, aber das Vorhandensein oder die Hinzufügung von einem bzw. einer oder mehreren Merkmalen, ganzen Zahlen, Schritten, Arbeitsabläufen, Elementen, Komponenten und/oder Gruppen davon nicht ausschließen.

Solange nichts anderes definiert ist, haben sämtliche hierin verwendeten Begriffe (einschließlich von technischen und wissenschaftlichen Begriffen) die gleiche Bedeutung, die ihnen ein Durchschnittsfachmann auf dem Gebiet, zu dem die Ausführungsbeispiele gehören, beimisst. Ferner sei klargestellt, dass Ausdrücke, z.B. diejenigen, die in allgemein verwendeten Wörterbüchern definiert sind, so zu interpretieren sind, als hätten sie die Bedeutung, die mit ihrer Bedeutung im Kontext der einschlägigen Technik konsistent ist, und nicht in einem idealisierten oder übermäßig formalen Sinn zu interpretieren sind, solange dies hierin nicht ausdrücklich definiert ist.

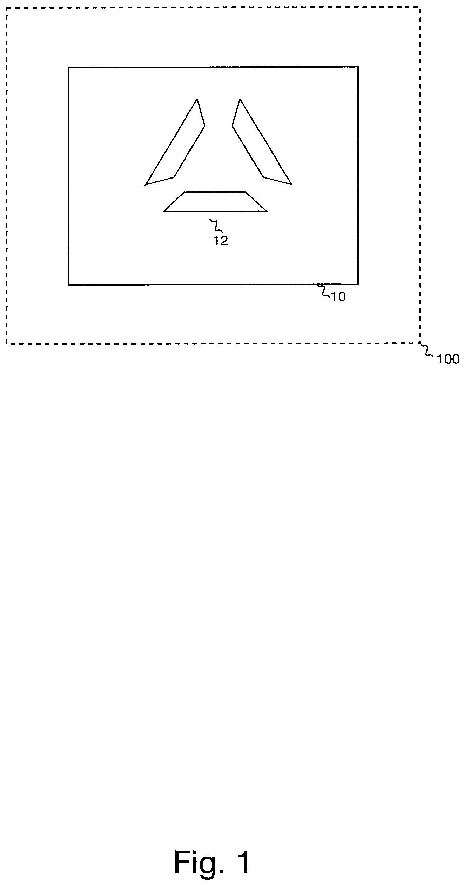

Fig. 1 zeigt ein Ausführungsbeispiel einer Vorrichtung 10 zur eindeutigen und individuellen Identifikation eines Substanzmessgerätes 100 eines Probanden, wobei die Vorrichtung 10 eine optische Kennung 12 umfasst. Ausführungsbeispiele schaffen auch ein Substanzmessgerät 100 mit der Vorrichtung 10. Die zu messende Substanz entspricht dabei einem Atemalkohol und/oder Betäubungsmitteln im Speichel des Probanden. Das Substanzmessgerät 100 ist demnach beispielsweise ein "mobiles" Atemalkoholmessgerät oder ein Messgerät zum Drogennachweis in Speichelproben. Die Bezeichnung "mobil" weist dabei darauf hin, dass das Gerät z.B. beim Homemonitoring einsetzbar ist, wobei das Gerät schnurgebunden oder auch per Batterie oder Akkumulator betreibbar sein kann.

Eindeutige und individuelle Identifikation meint dabei, dass das Substanzmessgerät 100 über die optische Kennung 12 wiedererkannt und von anderen Substanzmessgeräten unterschieden werden kann. Insofern kann die optische Kennung 12 zumindest in manchen Ausführungsbeispielen in Bezug auf das Substanzmessgerät 100 einzigartig sein, so dass es nur ein Substanzmessgerät 100 mit dieser optischen Kennung 12 gibt. Die optische Kennung 12 erschwert damit die Manipulierbarkeit, insbesondere durch Verwendung anderer ggf. gleicher Substanzmessgeräte. In einigen Ausführungsbeispielen kann somit verifiziert werden, dass ein bestimmtes, einem Probanden zur Verfügung gestelltes Substanzmessgerät 100 auch zur Probenaufnahme verwendet wurde. Die Vorrichtung 10 ist dann zur Verifikation einer Zuordnung zwischen dem Substanzmessgerät 100 und dem Probanden 300 ausgebildet. Die optische Kennung 12 ist daher ausgebildet, um das Substanzmessgerät 100 in Bildmaterial (Foto oder Video) wiedererkennbar und von anderen Substanzmessgeräten unterscheidbar zu machen. Dabei können verschiedenartige optische Kennungen verwendet werden, darunter können permanente Kennungen und/oder auch temporäre Kennungen bzw. Verifikationssignale verwendet werden, wie im Folgenden noch näher erläutert wird.

In dem Ausführungsbeispiel der Fig.1 ist die optische Kennung 12 eine geometrische Anordnung von mehreren Markierungen, in der Fig. 1 sind es drei Markierungen. Die geometrische Anordnung entspricht einer Anordnung innerhalb eines Dreiecks. Die geometrische Anordnung umfasst zumindest die drei Marker, die entlang der Seiten eines Dreiecks angeordnet sind. Die drei Marker sind in diesem Ausführungsbeispiel als reflektierende Folie ausgeführt. Demnach umfasst die optische Kennung 12 in diesem Ausführungsbeispiel ein oder mehrere Reflektoren und ein oder mehrere reflektierende Folienabschnitte.

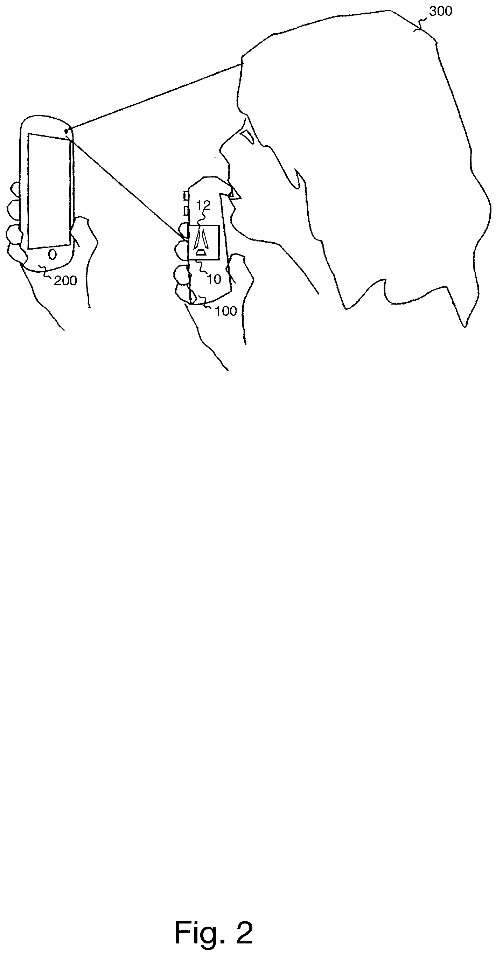

Fig. 2 zeigt ein weiteres Ausführungsbeispiel einer Vorrichtung 10 zur Identifikation eines Substanzmessgerätes 100 im Einsatz mit einem Probanden 300. Fig. 2 zeigt eine mobile Kamera 200, die beispielsweise in einem Mobilfunkgerät integriert ist. Die Kamera 200 ist ausgebildet, um den Probanden 300 während einer Substanzmessung mit dem Substanzmessgerät 100 zu fotografieren oder zu filmen, und damit gleichzeitig auch Bilddaten der optischen Kennung 12 der Vorrichtung 10 aufzunehmen. Beispielsweise kann die optische Kennung 12 mit Mitteln der digitalen Bildverarbeitung erkannt werden und somit festgestellt werden, dass das richtige Substanzmessgerät 100 in den Bilddaten abgebildet wurde.

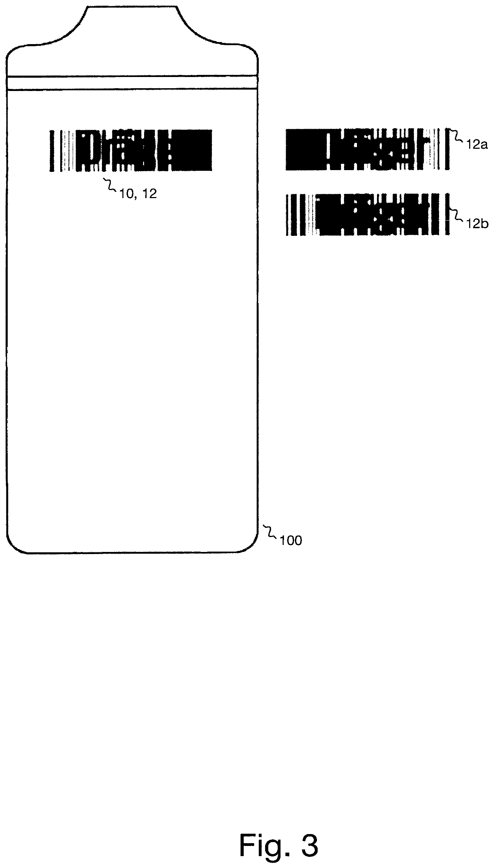

Hierin beschrieben (nicht Teil der Erfindung) sind ein oder mehrere Barcodes und/oder ein oder mehrere QR-Codes auf dem Gehäuse des Substanzmessgerätes 100. Fig. 3 zeigt Substanzmessgeräten 100 mit verschiedenen Kennungen 12, 12a, 12b, die als mit Barcodes überdruckte Schriftzüge, hier "Dräger", ausgeführt sind. Dabei bieten optische Codes (Bar, QR, usw.) beispielsweise eine Vielzahl von Erscheinungsbildern und auch die Möglichkeit eine eindeutige Kennung zu schaffen.

In manchen Aspekten umfasst die optische Kennung 12 eine eindeutige Kennung. Ein weiteres Beispiel für eine eindeutige Kennung ist neben statischen optischen Codes wie Bar-Codes und QR-Codes das Verwenden einer Sequenz von optischen Signalen, wie im Folgenden noch näher erläutert werden wird.

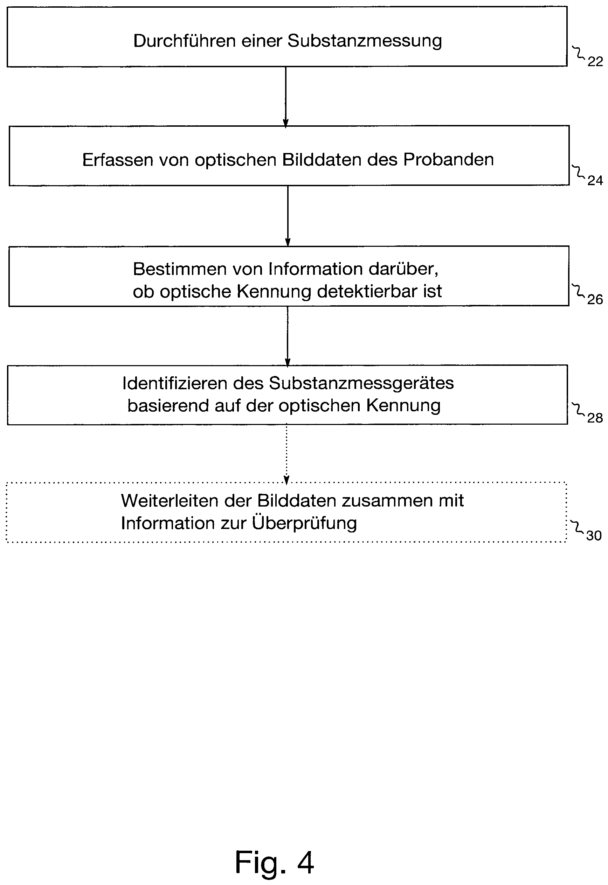

Fig. 4 zeigt ein Blockschaltbild eines Ausführungsbeispiels eines Ablaufdiagramms eines Verfahrens zur eindeutigen und individuellen Identifikation eines Substanzmessgerätes 100 eines Probanden 300, vgl. auch Fig. 2. Das Substanzmessgerät 100 weist eine optische Kennung 12 auf. Das Verfahren umfasst ein Durchführen 22 einer Substanzmessung durch den Probanden 300. Dabei erfolgt ein Erfassen 24 von optischen Bilddaten des Probanden 300 während der Substanzmessung zusammen mit dem Substanzmessgerät 100. Das Verfahren umfasst ferner ein Bestimmen 26 von Information darüber, ob die optische Kennung 12 in den Bilddaten detektierbar ist. Ein Identifizieren 28 des Substanzmessgerätes 100 erfolgt basierend auf der optischen Kennung 12. Wie die Fig. 4 weiter zeigt, kann optional auch ein Weiterleiten 30 der Bilddaten zusammen mit der Information zur Überprüfung erfolgen.

Die mit der Kamera 200 aufgenommenen Bilddaten können dann verarbeitet werden und eine Detektion der optischen Kennung 12 kann durchgeführt werden. Die Kamera 200 kann demnach ausgebildet sein, um zumindest eines der oben beschriebenen Verfahren durchzuführen. Die Kamera 200 kann ferner in ein Mobilfunkgerät integriert sein. In einem weiteren Beispiel umfasst die Kamera ferner einen Speicher (z.B. ein digitaler Speicher der volatil oder nicht-volatil sein kann) und ist ausgebildet, um die Bilddaten zusammen mit der Information abzuspeichern. Die Vorrichtung 10, die Kamera 200 bzw. das Mobilfunkgerät kann einer Hardwarekomponente entsprechen oder eine programmierbare Hardwarekomponente umfassen, z.B. einen Prozessor, Mikrokontroller, etc., der zur Ausführung entsprechend angepasster Software ausgebildet ist.

Weiterhin beschrieben ist daher ein Programm oder Computerprogramm mit einem Programmcode zur Durchführung eines der hierin beschriebenen Verfahren, wenn der Programmcode auf einem Computer, einem Prozessor oder einer programmierbaren Hardwarekomponente ausgeführt wird.

In manchen Beispielen kann demnach per Software in den Bilddaten oder den Fotos eine Information darüber eingebracht werden (Kennzeichnung), dass das Subtanzmessgerät 100 (z.B. Atemalkoholgerät) bzw. die optische Kennung 12 nicht vorhanden ist. Durch diese Kennzeichnung können Stichproben effizienter ausgewählt werden. Verstöße, in denen andere Personen für den Probegeber den Atemalkoholtest abgeben, können einfacher identifiziert werden. Darüber hinaus kann die Manipulation mit einem neu gekauften Gerät oder einem Nachbau erschwert oder sogar verhindert werden, wobei dann auf dem Bild die richtige Person mit dem "Neugerät" oder dem Nachbau ohne oder mit falscher Kennung abgebildet ist, allerdings in ein anderes Gerät gepustet wird.

In einem Beispiel wird eine Kombination, die insgesamt auch "Alcotester" genannt wird, aus Smartphone (Mobilfunkgerät) und Alkoholmessgerät 100 verwendet. Das Smartphone besitzt eine Kamera 200. Auf der Rückseite des Atemalkoholmessgeräts 100 werden drei stark reflektierende Folienabschnitte (Marker) im oberen Bereich angebracht, vgl. Fign. 1, 2. In diesem Ausführungsbeispiel sind sie in einem Dreieck zueinander mit einem Mindestabstand von 2cm zueinander angeordnet. Während der Probegabe wird der Blitz der Smartphonekamera 200 ausgelöst. Durch das helle Licht werden die Marker angeleuchtet, die dadurch wiederum durch die starke Reflektion von der Kamera 200 des Smartphones wahrgenommen werden. Durch die App des Smartphones wird ausgewertet, ob die definierten Marker während des Atemalkoholtests sichtbar waren. Dadurch kann nachgewiesen werden, dass der tatsächliche Probegeber 300 auch die Person auf dem Foto ist, bzw. dass das die Probe empfangende Gerät zur Zeit der Probenabgabe auch auf dem Foto ist. Sollte dies nicht der Fall sein, wird das Ergebnis des Atemalkoholtests mit einem auffälligen Vermerk versehen. Dieser kann bei den Stichproben priorisiert betrachtet werden. Somit können Verstöße vereinfacht und deutlich effizienter identifiziert werden, vgl. auch Fig. 2. Darüber hinaus kann in manchen Ausführungsbeispielen ferner überprüft werden, ob die Person auf dem Foto auch die Sollperson ist, beispielsweise durch eine Gesichtserkennung/Personen-Identifikations-Software. In manchen Ausführungsbeispielen kann dadurch verhindert oder erkannt werden, dass zwar eine Atemprobe in das richtige Gerät eingegangen ist, diese aber von der falschen Person abgegeben wurde.

Darüber hinaus kann die Manipulation mit dem "Neugerät" verhindert werden, indem die Folienabschnitte mit einem individuellen Muster versehen werden, welches nicht kopiert werden kann, beispielsweise mit Hologrammen, Wasserzeichen etc. Der Atemalkoholtester wird somit zum Unikat, vgl. Fig. 3. Das Muster wird bei Aufnahme des Homemonitoring-Programms in der App als Referenz hinterlegt. Das individuelle Muster (optische Kennung 12) kann in Form eines Bildabgleichs mit Hilfe der App und der Kamera 200 des Smartphones bei der Probegabe erkannt werden. Sollte ein Neugerät beschafft werden, würden die Muster nicht übereinstimmen und ein Verstoß gemeldet werden.

Anstelle von reflektierenden Folienabschnitten können auch Lichtquellen verwendet werden. In einem solchen Beispiel ist die optische Kennung 12 eine Sequenz optischer Signale, die durch ein oder mehre an dem Substanzmessgerät 100 angeordnete Lichtquellen erzeugbar ist. Die Lichtquellen können dabei auch einem Display also einer Art Bildschirm/Anzeige zur Darstellung von Grafiken entsprechen. In manchen Aspekten sind die Lichtquellen ausgebildet, um Licht in einem nicht sichtbaren Bereich zu emittieren, beispielsweise um Licht in einem Infrarotbereich (IR) zu emittieren. Dies kann Vorteile bieten, da z.B. ein schwarzes Gehäuse verwendet werden kann, durch das im IR-Bereich immer noch hindurchgeleuchtet werden kann, das im IR-Bereich demnach immer noch transparent oder teiltransparent ist. Beispielsweise können IR-LED's (Licht Emittierende Dioden) auf der Rückseite des Atemalkoholmessgeräts 100 verbaut werden oder auch eine einfache LED (z.B. zur Emission im sichtbaren Bereich). In beiden Fällen kann ein individuelles Blinkmuster weitere Manipulationen ausschließen.

Beispielsweise kann zwischen dem Mobilfunkgerät und dem Substanzmessgerät 100 eine Kommunikationsverbindung aufgebaut werden. Dafür sind beliebige schnurlose oder auch schnurgebundene Schnittstellen denkbar. Einige Beispiele sind kabelgebundene Kommunikation über Universal Serial Bus (USB) sowie schnurlose Kommunikation über Bluetooth, Near Field Communication (NFC) oder Wireless Local Area Network (WLAN).

Über eine solche Kommunikationsverbindung kann dann die App des Smartphones die ein oder mehreren Lichtquellen des Substanzmessgerätes 100 ansteuern und über die Kamera 200 gleichzeitig die Reaktion der Lichtquellen optisch erfassen. Damit kann eine Sequenz oder auch ein dargestellter Code (beispielsweise ein auf einem Display eingeblendeter Bar- oder QR Code) auch mit einer Zufallskomponente versehen werden sowie eine gerätespezifische Kennung des Substanzmessgerätes 100, sodass unter Kontrolle der App auch verschiedene Codes verwendet werden können. Dies kann die Sicherheit bzw. die Detektion von Täuschungsversuchen weiter erhöhen.

Die eindeutige und individuelle Identifikation erfolgt dann durch die Erfassung der Sequenz, die auch das Ergebnis einer Kombination von Sequenzen sein kann. Beispielsweise kann eine erste Sequenz, die dem Substanzmessgerät 100 zugeordnet ist, mit einer zweiten Sequenz, die dem Smartphone oder der Kamera 200 zugeordnet ist, kombiniert werden. In einigen Bei-spielen kann ergänzend oder alternativ noch eine Kombination mit einer Zufallssequenz, die individuell bei jeder Identifikation neu ermittelt wird, erfolgen. Insofern ist die eindeutige und individuelle Identifizierbarkeit zumindest temporär gegeben und damit die Verifikation der Zuordnung oder Verknüpfung ermöglicht. Im Falle von binären Sequenzen ist eine Übermittlung einer ersten Sequenz von dem Mobilgerät 200 an das Substanzmessgerät 100 denkbar, eine Verknüpfung der empfangenen Sequenz mit einer dem Substanzmessgerät 100 zugeordneten Sequenz (z.B. eine Xor-Verknüpfung, exklusives oder) und dann einer Ansteuerung der Lichtquellen mit der Ergebnissequenz. Über die Kamera kann dann die Ergebnissequenz erfasst und in dem Mobilgerät 200 verifiziert werden. Anhand der Ergebnissequenz kann die Identität des Substanzmessgerätes und damit die Zuordnung zum Probanden 300 verifiziert werden. Eine Erschwerung der Reproduzierbarkeit der optischen Kennung kann durch zusätzliche Kombination mit einer Zufallssequenz erreicht werden.

Aspekte basieren ferner auf der Erkenntnis, dass ein solches Identifikationsverfahren von einer Funktion der Kamera 200 bzw. des Mobilfunkgerätes abhängig sein kann. Darüber hinaus kann ein Mobilfunkgerät speziell für diesen Zweck ausgelegt sein und ggf. auch in seinen weiteren Funktion entsprechend eingeschränkt. Solche Einschränkungen können zu einer verminderten Alltagstauglichkeit eines solchen Mobilfunkgerätes führen, was ein Interesse zur sonstigen Nutzung einschränkt. Ein weiteres Ausführungsbeispiel ist ein Mobilfunkgerät, das in seiner Funktion für die Identifikation des Substanzmessgerätes 100 mit der Kamera 200 ausgebildet ist und in seinen weiteren Funktionen eingeschränkt ist. Beispielsweise kann das Mobilfunkgerät ausgebildet sein, um Anrufe zu nicht mehr als drei, fünf oder zehn vordefinierten Rufnummern durchführen zu können. Solche Anrufe können beispielsweise einen Notruf, einen Bewährungshelfer und eine frei wählbare Rufnummer bzw. eine Servicenummer des Betreibers umfassen.

Je nach bestimmten Implementierungsanforderungen können Aspekte in Hardware oder in Software implementiert sein. Die Implementierung kann unter Verwendung eines digitalen Speichermediums, beispielsweise einer Floppy-Disk, einer DVD, einer Blu-Ray Disc, einer CD, eines ROM, eines PROM, eines EPROM, eines EEPROM oder eines FLASH-Speichers, einer Festplatte oder eines anderen magnetischen oder optischen Speichers durchgeführt werden, auf dem elektronisch lesbare Steuersignale gespeichert sind, die mit einer programmierbaren Hardwarekomponente derart zusammenwirken können oder zusammenwirken, dass das jeweilige Verfahren durchgeführt wird.

Eine programmierbare Hardwarekomponente kann durch einen Prozessor, einen Computerprozessor (CPU = Central Processing Unit), einen Grafikprozessor (GPU = Graphics Processing Unit), einen Computer, ein Computersystem, einen anwendungsspezifischen integrierten Schaltkreis (ASIC = Application-Specific Integrated Circuit), einen integrierten Schaltkreis (IC = Integrated Circuit), ein Ein-Chip-System (SOC = System on Chip), ein programmierbares Logikelement oder ein feldprogrammierbares Gatterarray mit einem Mikro-prozessor (FPGA = Field Programmable Gate Array) gebildet sein.

Das digitale Speichermedium kann daher maschinen- oder computerlesbar sein. Manche Aspekte umfassen also einen Datenträger, der elektronisch lesbare Steuersignale aufweist, die in der Lage sind, mit einem programmierbaren Computersystem oder einer programmierbaren Hardwarekomponente derart zusammenzuwirken, dass eines der hierin beschriebenen Verfahren durchgeführt wird. Ein Aspekt ist somit ein Datenträger (oder ein digitales Speichermedium oder ein computerlesbares Medium), auf dem das Programm zum Durchführen eines der hierin beschriebenen Verfahren aufgezeichnet ist.

Allgemein können Ausführungsbeispiele der vorliegenden Erfindung als Programm, Firmware, Computerprogramm oder Computerprogrammprodukt mit einem Programmcode oder als Daten implementiert sein, wobei der Programmcode oder die Daten dahin gehend wirksam ist bzw. sind, eines der Verfahren durchzuführen, wenn das Programm auf einem Prozessor oder einer programmierbaren Hardwarekomponente abläuft. Der Programmcode oder die Daten kann bzw. können beispielsweise auch auf einem maschinenlesbaren Träger oder Datenträger gespeichert sein. Der Programmcode oder die Daten können unter anderem als Quellcode, Maschinencode oder Bytecode sowie als anderer Zwischencode vorliegen.

Ein weiteres Aspekt ist ferner ein Datenstrom, eine Signalfolge oder eine Sequenz von Signalen, der bzw. die das Programm zum Durchführen eines der hierin beschriebenen Verfahren darstellt bzw. darstellen. Der Datenstrom, die Signalfolge oder die Sequenz von Signalen kann bzw. können beispielsweise dahin gehend konfiguriert sein, um über eine Datenkommunikationsverbindung, beispielsweise über das Internet oder ein anderes Netzwerk, transferiert zu werden.

Beschrieben sind so auch Daten repräsentierende Signalfolgen, die für eine Übersendung über ein Netzwerk oder eine Datenkommunikationsverbindung geeignet sind, wobei die Daten das Programm darstellen.

Ein Programm gemäß einem Ausführungsbeispiel kann eines der Verfahren während seiner Durchführung beispielsweise dadurch umsetzen, dass dieses Speicherstellen ausliest oder in diese ein Datum oder mehrere Daten hinein schreibt, wodurch gegebenenfalls Schaltvorgänge oder andere Vorgänge in Transistorstrukturen, in Verstärkerstrukturen oder in anderen elektrischen, optischen, magnetischen oder nach einem anderen Funktionsprinzip arbeitenden Bauteile hervorgerufen werden. Entsprechend können durch ein Auslesen einer Speicherstelle Daten, Werte, Sensorwerte oder andere Informationen von einem Programm erfasst, bestimmt oder gemessen werden. Ein Programm kann daher durch ein Auslesen von einer oder mehreren Speicherstellen Größen, Werte, Messgrößen und andere Informationen erfassen, bestimmen oder messen, sowie durch ein Schreiben in eine oder mehrere Speicherstellen eine Aktion bewirken, veranlassen oder durchführen sowie andere Geräte, Maschinen und Komponenten ansteuern.

## Patentansprüche

1. Vorrichtung (10) zur eindeutigen und individuellen Identifikation eines Substanzmessgerätes (100) eines Probanden (300), wobei die Vorrichtung (10) eine optische Kennung (12) umfasst, die ausgebildet ist zur Wiedererkennung des Substanzmessgerätes (100) und zur Verifikation einer Zuordnung zwischen dem Substanzmessgerät (100) und dem Probanden (300) wobei die optische Kennung (12) einer Anordnung innerhalb eines Dreiecks von mehreren Markierungen, Barcodes, und/oder "Quick Response"-Codes, QR, oder von einer Kombination mindestens einer Markierung mit mindestens einem Barcode und/oder mindestens einem QR-Code für ein Gehäuse des Substanzmessgerätes (100) entspricht.

2. Vorrichtung (10) gemäß Anspruch 1, wobei die Anordnung innenhalb eines Dreiecks zumindest drei Marker umfasst, die entlang der Seiten des Dreiecks angeordnet sind.

3. Vorrichtung (10) gemäß einem der vorangehenden Ansprüche, wobei die optische Kennung (12) ein oder mehrere Reflektoren umfasst.

4. Vorrichtung (10) gemäß einem der vorangehenden Ansprüche, wobei die optische Kennung (12) ein oder mehrere reflektierende Folienabschnitte umfasst.

5. Vorrichtung (10) gemäß einem der vorangehenden Ansprüche, wobei die optische Kennung (12) eine eindeutige Kennung umfasst.

6. Vorrichtung (10) gemäß einem der vorangehenden Ansprüche, wobei die optische Kennung (12) eine Sequenz optischer Signale umfasst, die durch ein oder mehrere an dem Substanzmessgerät (100) angeordnete Lichtquellen erzeugbar ist.

7. Vorrichtung (10) gemäß Anspruch 6, wobei die Lichtquellen ausgebildet sind, um Licht in einem nicht sichtbaren Bereich zu emittieren.

8. Substanzmessgerät (100) mit einer Vorrichtung (10) gemäß einem der vorangehenden Ansprüche.

9. Verfahren zur eindeutigen und individuellen Identifikation eines Substanzmessgerätes (100) eines Probanden (300), wobei das Substanzmessgerät (100) eine optische Kennung (12) aufweist, mit den Verfahrensschritten Durchführen (22) einer Substanzmessung durch den Probanden (300);
Erfassen (24) von optischen Bilddaten des Probanden (300) während der Substanzmessung zusammen mit dem Substanzmessgerät (100);
Bestimmen (26) von Information darüber, ob die optische Kennung (12) in den Bilddaten detektierbar ist;
Identifizieren (28) und Wiedererkennen des Substanzmessgerätes (100) basierend auf der optischen Kennung (12) und
Verifizieren einer Zuordnung zwischen dem Substanzmessgerät (100) und dem Probanden (300)
wobei die optische Kennung (12) verwendet wird, die einer Anordnung innerhalb eines Dreiecks von mehreren Markierungen, Barcodes, und/oder "Quick Response"-Codes, QR, oder von einer Kombination mindestens einer Markierung mit mindestens einem Barcode und/oder mindestens einem QR-Code für ein Gehäuse des Substanzmessgerätes (100) entspricht.

10. Verfahren gemäß Anspruch 9, ferner umfassend
Weiterleiten der Bilddaten zusammen mit der Information zur Überprüfung.

11. Programm mit einem Programmcode zur Durchführung eines der Verfahren der Ansprüche 9 oder 10, wenn der Programmcode auf einem Computer, einem Prozessor oder einer programmierbaren Hardwarekomponente ausgeführt wird.

## Claims

1. Device (10) for the unique and individual identification of a substance measuring instrument (100) of a test subject (300), wherein the device (10) comprises an optical identifier (12) which is designed to recognize the substance measuring instrument (100) and to verify an association between the substance measuring instrument (100) and the test subject (300), wherein the optical identifier (12) corresponds to an arrangement within a triangle of a plurality of markings, barcodes, and/or "quick response" codes, QR, or of a combination of at least one marking with at least one barcode and/or at least one QR code for a housing of the substance measuring instrument (100).

2. Device (10) according to claim 1, wherein the arrangement within a triangle comprises at least three markers arranged along the sides of the triangle.

3. Device (10) according to either of the preceding claims, wherein the optical identifier (12) comprises one or more reflectors.

4. Device (10) according to any of the preceding claims, wherein the optical identifier (12) comprises one or more reflective film portions.

5. Device (10) according to any of the preceding claims, wherein the optical identifier (12) comprises a unique identifier.

6. Device (10) according to any of the preceding claims, wherein the optical identifier (12) comprises a sequence of optical signals that can be generated by one or a plurality of light sources arranged on the substance measuring instrument (100).

7. Device (10) according to claim 6, wherein the light sources are designed to emit light in a non-visible region.

8. Substance measuring instrument (100) having a device (10) according to any of the preceding claims.

9. Method for the unique and individual identification of a substance measuring instrument (100) of a test subject (300), wherein the substance measuring instrument (100) has an optical identifier (12), comprising the method steps of carrying out (22) a substance measurement by the test subject (300);
recording (24) optical image data of the test subject (300) during the substance measurement together with the substance measuring instrument (100);
determining (26) information as to whether the optical identifier (12) is detectable in the image data;
identifying (28) and recognizing the substance measuring instrument (100) based on the optical identifier (12) and
verifying an association between the substance measuring instrument (100) and the test subject (300)
wherein the optical identifier (12) is used which corresponds to an arrangement within a triangle of a plurality of markings, barcodes, and/or "quick response" codes, QR, or of a combination of at least one marking with at least one barcode and/or at least one QR code for a housing of the substance measuring instrument (100).

10. Method according to claim 9, further comprising forwarding the image data together with the information for review.

11. Program having a program code for carrying out a method according to claim 9 or 10 when the program code is executed on a computer, a processor, or a programmable hardware component.

## Revendications

1. Dispositif (10) pour l'identification unique et individuelle d'un appareil de mesure de substance (100) d'un sujet (300), dans lequel le dispositif (10) comprend un signe identificateur optique (12) qui est conçu pour la reconnaissance de l'appareil de mesure de substance (100) et pour la vérification d'une association entre l'appareil de mesure de substance (100) et le sujet (300), dans lequel le signe identificateur optique (12) correspond à une disposition à l'intérieur d'un triangle de plusieurs marquages, codes à barres, et/ou codes « à réponse rapide », QR, ou d'une combinaison d'au moins un marquage avec au moins un code à barres et/ou au moins un code QR pour un boîtier de l'appareil de mesure de substance (100).

2. Dispositif (10) selon la revendication 1, dans lequel l'agencement comprend, à l'intérieur d'un triangle, au moins trois marqueurs disposés le long des côtés du triangle.

3. Dispositif (10) selon l'une des revendications précédentes, dans lequel le signe identificateur optique (12) comprend un ou plusieurs réflecteurs.

4. Dispositif (10) selon l'une des revendications précédentes, dans lequel le signe identificateur optique (12) comprend une ou plusieurs sections de film réfléchissant.

5. Dispositif (10) selon l'une des revendications précédentes, dans lequel le signe identificateur optique (12) comprend un signe identificateur unique.

6. Dispositif (10) selon l'une des revendications précédentes, dans lequel le signe identificateur optique (12) comprend une séquence de signaux optiques qui peuvent être générés par une ou plusieurs sources lumineuses disposées sur l'appareil de mesure de substance (100).

7. Dispositif (10) selon la revendication 6, dans lequel les sources lumineuses sont réalisées pour émettre de la lumière dans une plage non visible.

8. Appareil de mesure de substance (100) comportant un dispositif (10) selon l'une des revendications précédentes.

9. Procédé pour l'identification unique et individuelle d'un appareil de mesure de substance (100) d'un sujet (300), dans lequel l'appareil de mesure de substance (100) présente un signe identificateur optique (12), comportant les étapes de procédé consistant à réaliser (22) une mesure de substance par le sujet (300) ;
détecter (24) des données d'image optique du sujet (300) pendant la mesure de substance conjointement avec l'appareil de mesure de substance (100) ;
déterminer (26) des informations concernant le fait que le signe identificateur optique (12) est détectable dans les données d'image ou non ;
identifier (28) et reconnaître l'appareil de mesure de substance (100) sur la base du signe identificateur optique (12) et
vérifier une association entre l'appareil de mesure de substance (100) et le sujet (300)
dans lequel le signe identificateur optique (12) est utilisé, lequel correspond à une disposition à l'intérieur d'un triangle de plusieurs marquages, codes à barres, et/ou codes « à réponse rapide », QR, ou d'une combinaison d'au moins un marquage avec au moins un code à barres et/ou au moins un code QR pour un boîtier de l'appareil de mesure de substance (100).

10. Procédé selon la revendication 9, comprenant en outre la transmission des données d'image conjointement avec les informations pour la vérification.

11. Programme comportant un code de programme pour la réalisation de l'un des procédés selon la revendication 9 ou 10, lorsque le code de programme est exécuté sur un ordinateur, un processeur ou un composant matériel programmable.
